# FASCICULE DE BREVET EUROPEEN

(11) **EP 3 881 884 B1**
(45) Date de publication et mention de la délivrance du brevet: **21.08.2024**
(21) Numéro de dépôt: 21160656.1
(22) Date de dépôt: 04.03.2021
(51) Int. Cl.: B01D 46/52, A61M 16/00, F04D 25/08, F04D 29/58, F04D 29/70, B01D 46/00

(54) **VENTILATEUR MÉDICAL À ENTRÉES D'AIR PROTÉGÉES PAR DES FILTRES AGENCÉS DANS UNE CASSETTE DE FILTRATION COMMUNE DÉTACHABLE**
MEDIZINISCHES BELÜFTUNGSGERÄT MIT LUFTEINLÄSSEN, DIE DURCH FILTER GESCHÜTZT SIND, DIE IN EINER GEMEINSAMEN ABNEHMBAREN FILTERKASSETTE ANGEORDNET SIND
MEDICAL FAN WITH AIR INPUT PROTECTED BY FILTERS ARRANGED IN A DETACHABLE COMMON FILTERING CASSETTE

(30) Priorité: 18.03.2020 FR 2002670
(43) Date de publication de la demande: 22.09.2021
(73) Titulaire: Air Liquide Medical Systems, 92160 Antony (FR)
(72) Inventeur: DAVOINE, Romain, 38360 Sassenage (FR); GIARD, Pauline, 92160 Antony (FR); LEBATTEUR, Nicolas, 92160 Antony (FR)
(74) Mandataire: Air Liquide

(56) Documents cités:
- WO-A1-2018/074935
- WO-A1-2018/158387
- DE-A1- 102014 009 895
- FR-A3- 3 067 251
- US-A1- 2008 178 879

## Description

L'invention concerne un ventilateur médical dont les entrées d'air patient du circuit d'air patient et d'air de refroidissement du circuit d'air de refroidissement sont équipées de filtres portés par une cassette de filtration commune fixée de manière détachable, i.e. amovible, afin de pouvoir être montée et démontée aisément depuis l'extérieur du ventilateur, c'est-à-dire sans avoir à ouvrir la carcasse du ventilateur ou à intervenir à l'intérieur de celle-ci, ainsi qu'une telle cassette de filtration.

Dans un hôpital ou sur le terrain, le traitement d'un patient en insuffisance respiratoire, en urgence ou non, peut s'effectuer à l'aide d'un appareil d'assistance respiratoire ou ventilateur médical fournissant de l'air, éventuellement additionné d'oxygène, c'est-à-dire de l'air enrichi en oxygène.

Le ventilateur médical doit permettre la ventilation d'un patient dans des environnements « normaux », par exemple dans un bâtiment, dans une ville, sur une route de campagne ou autres, mais aussi dans des environnements « difficiles », notamment des environnements contaminés par des polluants, tels que poussières, pollens, bactéries ou autres, ou dans des environnements pouvant être chauds, par exemple en plein été ou dans des régions désertiques ou tropicales.

Afin d'assurer un niveau de sécurité et protection du patient, lors de son traitement par administration d'air ou d'air enrichi en oxygène, il est nécessaire de pouvoir filtrer l'air ambiant rentrant dans le ventilateur afin de le débarrasser de ses polluants atmosphériques avant de l'administrer au patient.

En outre, un ventilateur médical est amené à chauffer du fait de sa composition électromécanique, notamment du fait de la présence de composants électroniques, tels qu'écran numérique, micro-soufflante à moteur électrique, carte(s) électronique(s) à microprocesseur(s), batterie(s) internes..., cet échauffement pouvant être accentué dans les milieux chauds.

De là, il est nécessaire de refroidir le ventilateur et ceci se fait couramment en balayant d'intérieur du ventilateur avec flux d'air de refroidissement servant à évacuer les calories vers l'extérieur. Mais, là encore, pour éviter la contamination de l'intérieur du ventilateur médical par des polluants atmosphériques, il convient de filtrer aussi l'air de refroidissement servant au refroidissement du ventilateur médical.

Pour ce faire, les ventilateurs médicaux sont souvent équipés de filtres agencés sur le circuit de gaz respiratoire et sur le circuit d'air de refroidissement de ces ventilateurs. Or, remplacer ces filtres est compliqué et fastidieux car ils sont agencés dans la carcasse des ventilateurs, ce qui oblige à un démontage du ventilateur pour permettre à un opérateur de maintenance de les atteindre et de les changer lorsqu'ils sont saturés.

DE-A-10 2014 009 895 propose un appareil respiratoire comprenant deux entrées de gaz distinctes, munies chacune d'un filtre. Il n'est pas clairement expliqué comment procéder pour les remplacer mais, au vu de l'architecture de cet appareil, chaque filtre doit être extrait et changé indépendamment de l'autre.

Ces opérations de démontage/remontage sont d'autant plus fastidieuses que les remplacements sont fréquents. Ainsi, selon les environnements dans lesquels les ventilateurs sont utilisés, le remplacement des filtres doit parfois avoir lieux plusieurs fois par an.

Le problème est de proposer un ventilateur médical d'architecture simplifiée, en particulier configuré pour permettre un remplacement aisé et rapide des filtres à air protégeant le circuit de gaz respiratoire et sur le circuit d'air de refroidissement du ventilateur médical.

La solution concerne un ventilateur médical comprenant une micro-soufflante motorisée et des composants électroniques, et comprenant en outre :
- un circuit d'air patient reliant fluidiquement une entrée d'air patient à une entrée d'aspiration de la micro-soufflante pour alimenter la micro-soufflante avec de l'air, et
- un circuit d'air de refroidissement comprenant une entrée d'air de refroidissement pour acheminer de l'air de refroidissement destinée à refroidir par échange thermique au moins une partie des composants électroniques,
et dans lequel l'entrée d'air patient du circuit d'air patient est équipée d'un premier filtre, et l'entrée d'air de refroidissement du circuit d'air de refroidissement est équipée d'un second filtre.

Selon l'invention, les premier et second filtres sont portés par une cassette de filtration commune détachable (i.e. amovible).

Selon le mode de réalisation considéré, le ventilateur médical de l'invention peut comprendre l'une ou plusieurs des caractéristiques suivantes :
- la cassette de filtration commune détachable est configurée pour pouvoir être montée et démontée depuis l'extérieur du ventilateur médical, notamment en vue d'un entretien ou d'un remplacement des filtres.
- lesdits premier et second filtres sont solidarisés à la cassette de filtration de sorte de pouvoir être extraits ou insérés, i.e. mis en place, simultanément dans le ventilateur médical lors du retrait ou de l'insertion, respectivement, de la cassette de filtration commune au sein du ventilateur médical.
- le circuit d'air patient et le circuit d'air de refroidissement comprennent des passages ou conduits de gaz, ou analogues.
- il comprend une carcasse externe rigide.
- la carcasse du ventilateur médical est en polymère.
- la micro-soufflante motorisée et les composants électroniques sont agencés dans la carcasse du ventilateur.
- la cassette de filtration commune comprenant les premier et second filtres est fixée de manière détachable au ventilateur.
- la cassette de filtration commune comprenant les premier et second filtres est fixée de manière détachable à la carcasse du ventilateur.
- la cassette de filtration est configurée pour se fixer, de manière détachable, au ventilateur au niveau de l'entrée d'air patient du circuit d'air patient et de l'entrée d'air de refroidissement du circuit d'air de refroidissement, de préférence pour se fixer à la carcasse du ventilateur.
- l'entrée d'air patient du circuit d'air patient et l'entrée d'air de refroidissement du circuit d'air de refroidissement sont situées dans au moins un compartiment d'entrée ouvert vers l'extérieur, c'est-à-dire présentant une ouverture communiquant vers l'extérieur du ventilateur.
- il comprend des moyens de mise en circulation de l'air de refroidissement comme par exemple un ventilateur de refroidissement et/ou la convection naturelle et/ou des déflecteurs pour orienter l'air de refroidissement.
- le compartiment d'entrée est aménagé dans la carcasse du ventilateur médical.
- le compartiment d'entrée est configuré pour loger la cassette de filtration comportant les premier et second filtres.
- le compartiment d'entrée comprend une paroi de séparation interne définissant une première et une seconde chambre.
- le premier filtre vient se loger dans la première chambre et le second filtre vient se loger dans la seconde chambre, lorsque la cassette de filtration est insérée dans le compartiment d'entrée.
- la cassette de filtration comprend un espacement séparant les premier filtre et second filtre, la paroi de séparation interne du compartiment d'entrée du ventilateur venant se loger dans l'espacement de la cassette de filtration, lorsque ladite cassette de filtration est insérée dans le compartiment d'entrée du ventilateur.
- la cassette de filtration comprend un cadre-support en polymère portant les premier filtre et second filtre.
- la cassette de filtration comprend des moyens de fixation permettant de la fixer de manière détachable au ventilateur, par exemple les moyens de fixation comprennent des clips incorporés à la cassette de filtration, des vis ou analogue de fixation ou un filetage.
- le circuit d'air de refroidissement est configuré pour acheminer de l'air de refroidissement pour refroidir par échange thermique (direct ou indirect) un ou plusieurs composants électroniques choisis parmi une carte électronique à microprocesseur(s), un écran d'IHM ou interface homme-machine, une batterie interne ou un moteur de micro-soufflante.
- le circuit d'air de refroidissement est configuré pour opérer un balayage gazeux avec un flux d'air de refroidissement d'un ou plusieurs composants électroniques du ventilateur de manière à obtenir un échange thermique (i.e. un transfert de calories) entre le flux d'air de refroidissement et le ou les composants électroniques à refroidir.
- la micro-soufflante, aussi appelée turbine ou compresseur, comprend un moteur électrique.
- la micro-soufflante comprend un carter de protection protégeant le rotor et le stator.
- la micro-soufflante est logée dans un boitier interne agencé dans la carcasse du ventilateur.
- la carte électronique à microprocesseur(s) est configurée pour piloter la micro-soufflante.
- la (ou les) batterie interne est rechargeable.
- la (ou les) batterie interne alimente en courant électrique, au moins la micro-soufflante, la carte électronique et l'écran de l'IHM.
- les premier filtre et second filtre sont identiques ou différents.
- les premier filtre et second filtre sont choisis pour stopper les polluants, i.e. particules, ayant une taille supérieure ou égale à 0.3 µm.
- les premier filtre et second filtre sont des filtres à air à haute efficacité, c'est-à-dire des filtres de type HEPA.
- la micro-soufflante comprend une sortie de gaz alimentant fluidiquement un circuit de fourniture de gaz au patient, notamment d'air ou de mélange air/O₂ débouchant au niveau un raccord de sortie de gaz.
- le circuit d'air de refroidissement comprend au moins une sortie d'évacuation d'air chaud permettant d'évacuer vers l'atmosphère ambiante, l'air ayant été réchauffé par échange thermique (i.e. échange de calories) avec les composants électroniques du ventilateur.

L'invention porte aussi sur une cassette de filtration pour un ventilateur médical selon la technologie, caractérisée en ce qu'elle comprend un cadre-support portant les premier et second filtres, lesdits premier et second filtres étant espacés l'un de l'autre par un espacement.

Selon le mode de réalisation considéré, la cassette de filtration de la technologie peut comprendre l'une ou plusieurs des caractéristiques suivantes :
- le cadre-support est en polymère, par exemple en thermoplastique, notamment de type ABS, ABS-PC ou PLA.
- l'espacement a une forme de fente, c'est-à-dire un espacement de forme allongée.
- le cadre-support comprend une paroi externe traversée par des orifices de passage d'air, par exemple des orifices circulaires ou analogues, des fentes ou autres.
- le premier filtre a une taille supérieure à celle du second filtre, de préférence une taille double.
- le premier et second filtres sont de préférence une forme parallélépipédique, en particulier parallélépipédique rectangle.
- le cadre-support comprend des moyens d'étanchéité fluidique, telle une lèvre, un joint ou analogue.
- le cadre-support comprend des moyens de fixation configurés pour permettre une fixation, i.e. solidarisation détachable, de la cassette de filtration au sein du ventilateur médical, par exemple par emboitement, clipsage, vissage ou autres.
- les premier et second filtres sont des filtres HEPA.
- les premier et second filtres sont configurés pour stopper les polluants, i.e. particules, ayant une taille supérieure ou égale à 0.3 µm.

L'invention va maintenant être mieux comprise grâce à la description détaillée suivante, faite à titre illustratif mais non limitatif, en référence aux figures annexées parmi lesquelles :
Fig. 1 schématise un ventilateur médical équipé d'une cassette de filtration selon l'invention,
Fig. 2 est une vue de face de la cassette de filtration insérée dans le ventilateur médical de la Fig. 1,
Fig. 3 est une autre vue du ventilateur médical de la Fig. 1,
Fig. 4 illustre le montage de la cassette de filtration dans le ventilateur médical de la Fig. 1,
Fig. 5 schématise la cassette de filtration et les circuits de gaz internes du ventilateur médical de la Fig. 1,
Fig. 6 est une vue de face de la cassette de filtration du ventilateur médical de la Fig. 5, et
Fig. 7 est une vue tridimensionnelle de la cassette de filtration insérée dans le ventilateur médical de la Fig. 1.

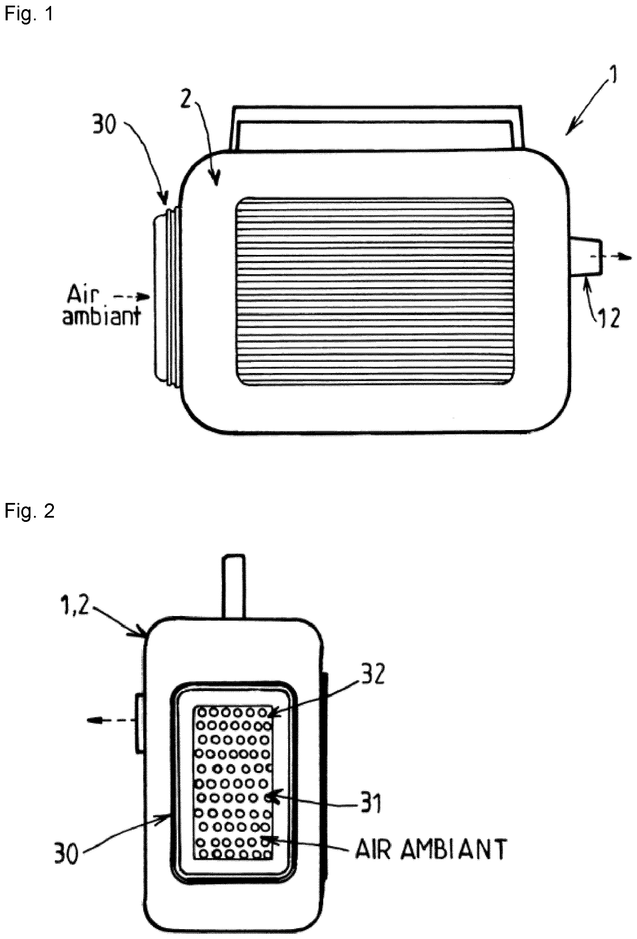

La Fig. 1 schématise un ventilateur médical 1, par exemple d'urgence, équipé d'une la cassette de filtration 30 comprenant les premier 31 et second 32 filtres selon l'invention. Comme illustré en Fig. 2, la cassette de filtration 30 vient s'insérer dans une des faces latérales de la carcasse 2 du ventilateur 1.

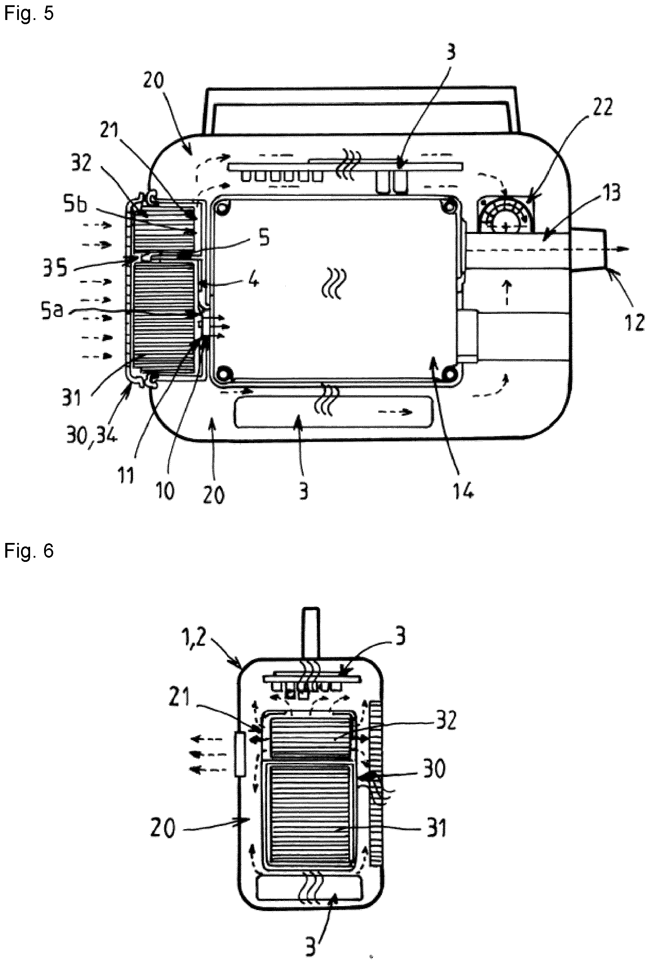

Plus précisément et schématisé en Fig. 5 et Fig. 6, le premier filtre 31 est agencé au niveau de l'entrée d'air patient 11 du circuit d'air patient 10 du ventilateur 1, alors que le second filtre 32 est agencé au niveau de l'entrée d'air de refroidissement 21 du circuit d'air de refroidissement 20 du ventilateur 1 en étant fixés l'un et l'autre de manière détachable, c'est-à-dire extractible, de manière à pouvoir être montés et démontés aisément et rapidement par un opérateur, directement depuis l'extérieur du ventilateur 1, c'est-à-dire sans qu'il n'ait à intervenir à l'intérieur de la carcasse 2 du ventilateur médical 1.

Sur les Fig. 1 à Fig. 6, les flux de gaz sont schématisés par des flèches en trait interrompu (- - - >).

Le premier filtre 31 peut être identique au second filtre 32 ou, le cas échéant, ils peuvent être différents l'un de l'autre, c'est-à-dire formés de média de filtration différents.

L'air ambiant peut être naturellement chargé de polluants atmosphériques, tels que particules, poussières, pollens, bactéries ou autre, qu'il est nécessaire de retenir afin de ne pas polluer les circuits d'air 10, 20 du ventilateur 1. Préférentiellement, le media de filtration des premier et second filtres 31, 32 a une capacité d'arrêt des polluants atmosphériques ayant des dimensions supérieures à 0,3 µm, par exemple des filtres à air de type HEPA (High Efficiency Particulate Air). Par exemple, les premier et second filtres 31, 32 peuvent être formés de papier.

Comme schématisé en Fig. 5, le ventilateur médical 1 comprend, agencés dans sa carcasse rigide externe 2, une micro-soufflante à moteur électrique (non visible) et d'autres composants électroniques 3 qui est nécessaire de refroidir pendant le fonctionnement du ventilateur médical 1, par exemple une carte électronique à microprocesseur, un écran d'IHM, une batterie interne et/ou le moteur de micro-soufflante.

Pour ce faire, on met en oeuvre un balayage gazeux avec de l'air de refroidissement amené par le circuit d'air de refroidissement 20 du ventilateur 1, lequel est configuré pour acheminer de l'air de refroidissement jusqu'au contact direct ou indirect des composants électroniques 3 à refroidir de manière à opérer un échange thermique entre l'air et ses composants électroniques 3, et à ainsi les refroidir par échange de calories.

Le circuit d'air de refroidissement 20 du ventilateur 1 comprend l'entrée d'air de refroidissement 21 au niveau de laquelle est inséré le second filtre 32.

La circulation d'air de refroidissement se fait via un ventilateur de refroidissement 22 en Fig.5 ou, selon le cas, par convection naturelle et/ou via des déflecteurs pour orienter le flux d'air.

Par ailleurs, il est prévu aussi un circuit d'air patient 10 qui relie fluidiquement l'entrée d'air patient 11 à une entrée d'aspiration de la micro-soufflante de manière à fournir de l'air à la micro-soufflante. L'air est aspiré par la micro-soufflante pendant son fonctionnement. L'air ressort par la sortie de gaz de la micro-soufflante et alimente fluidiquement un circuit de fourniture de gaz 13 au patient débouchant au niveau d'un raccord de sortie de gaz 12 agencé sur la carcasse 2 du ventilateur 1. Le gaz délivré par le raccord de sortie de gaz 12 du ventilateur 1 peut être de l'air ou un mélange air/O₂. Il est ensuite acheminé jusqu'au patient via un conduit flexible venant se raccorder fluidiquement au raccord de sortie de gaz 12 par l'une de ses extrémités, et comprenant à son autre extrémité, une interface respiratoire patient, tel qu'un masque respiratoire, servant à distribuer le gaz au patient.

Sur la Fig. 5, la micro-soufflante motorisée est agencée dans un caisson 14 traversé par une partie du circuit d'air patient 10.

La micro-soufflante motorisée et les autres composants électroniques 3 nécessitant du courant électrique pour fonctionner, notamment une carte électronique à microprocesseur commandant la micro-soufflante, sont alimentés en courant électrique par une (des) batterie, de préférence rechargeable.

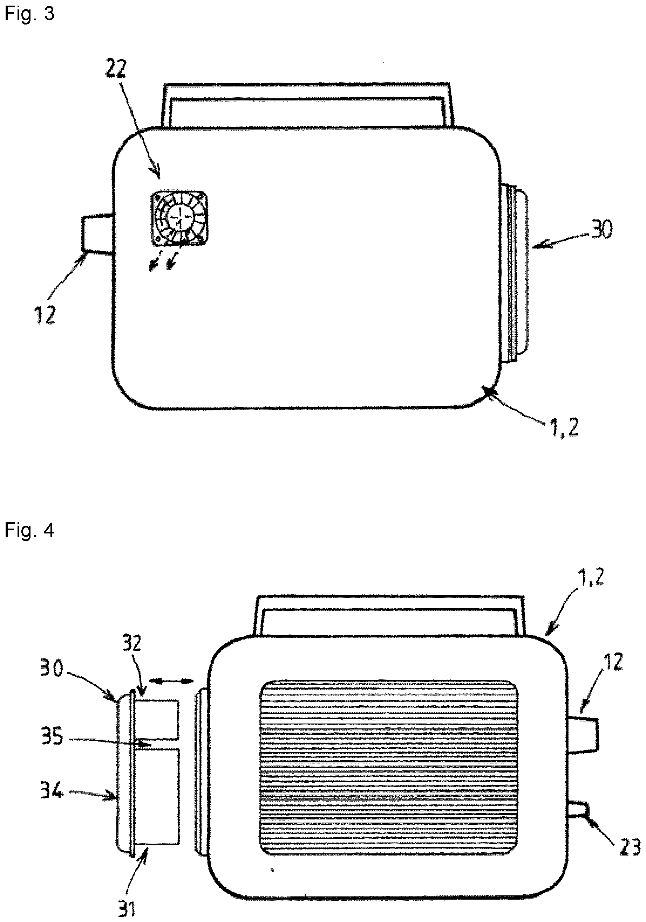

Préférentiellement, comme illustré en Fig. 4 à Fig. 7, les premier et second filtres 31, 32 sont portés par une cassette de filtration 30 commune qui est configurée pour se fixer, de manière détachable, au ventilateur 1 au niveau de l'entrée d'air patient 11 du circuit d'air patient 10 et de l'entrée d'air de refroidissement 21 du circuit d'air de refroidissement 20.

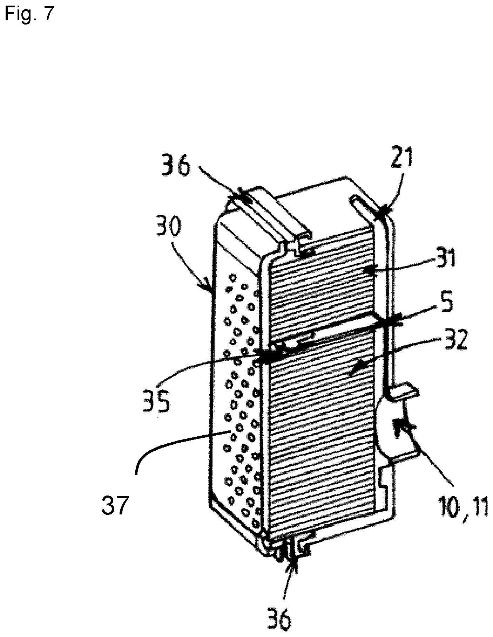

La cassette de filtration 30 peut se présenter sous la forme d'un cadre-support 34, par exemple en polymère, portant les premier et second filtres 31, 32, lesdits filtres étant séparés l'un de l'autre par un espacement 35, comme illustré en en Fig. 5 et Fig. 7. Par exemple, la cassette de filtration 30 peut être réalisée en thermoplastique, notamment de type ABS, ABS-PC ou PLA.

Par ailleurs, l'entrée d'air patient 11 du circuit d'air patient 10 et l'entrée d'air de refroidissement 21 du circuit d'air de refroidissement 20 se trouvent dans un compartiment d'entrée 4 ouvert vers l'extérieur, aménagé dans la carcasse 2 du ventilateur 1. Ce compartiment d'entrée 4 est configuré, i. conçu et dimensionné, pour loger, de manière extractible, la cassette de filtration 30 équipée des premier et second filtres 31, 32.

Plus précisément, le compartiment d'entrée 4 du ventilateur 1 comprend une paroi de séparation 5 interne définissant une première 5a et une seconde 5b chambre. Le premier filtre 31 vient se loger dans la première chambre 5a et le second filtre 32 vient se loger dans la seconde chambre 5b, lorsque la cassette de filtration 30 est insérée dans le compartiment d'entrée 4. Par ailleurs, la paroi de séparation 5 interne du compartiment d'entrée 4 vient alors se loger, quant à elle, dans l'espacement 35 de la cassette de filtration 30, comme montré sur la Fig. 5.

Les première et seconde chambres 5a, 5b ont dans le mode de réalisation présenté des tailles différentes car le premier filtre 31 a lui-aussi une taille supérieure à celle du second filtre 32 car la quantité d'air à fournir au circuit patient 10 est supérieure à celle à fournir au circuit de refroidissement 20.

Par exemple, le volume total occupé par les deux filtres 31, 32 peut être de l'ordre de 100 x 50 x 35 mm avec une répartition de 1/3 pour le second filtre 32 et de 2/3 pour le premier filtre 31, c'est-à-dire que le premier filtre 31 a une taille double de celle du second filtre 32. Ils peuvent avoir une forme approximativement parallélépipédique rectangle.

Le montage ou démontage de la cassette de filtration 30 est illustré sur la Fig. 4. Comme on le voit, la cassette de filtration 30 comprenant les premier et second filtres 31, 32 est insérée ou extraite facilement depuis l'extérieur du ventilateur 1, sans avoir à ouvrir la carcasse 2.

La fixation de la cassette de filtration 30 au sein du compartiment d'entrée 4 du ventilateur 1 est assurée par des moyens de fixation, par exemple emboitement, clipsage ou vissage. L'étanchéité fluidique est assurée par des moyens ou un dispositif d'étanchéité 36, tel un joint à lèvres ou analogue, par exemple un joint d'étanchéité en thermoplastique élastomère ou silicone, visibles sur Fig. 7.

Par ailleurs, le cadre-support 34 de la cassette de filtration 30 comprend une paroi externe 37 traversée par des orifices 38 de passage d'air permettant à l'air ambiant de passer au travers puis d'être filtré par les filtres 31, 32. La paroi externe 37 est dirigée vers l'extérieur lorsque la cassette de filtration 30 est insérée dans le compartiment d'entrée 4 du ventilateur 1. Cette paroi externe 37 forme alors un couvercle ou toit venant fermer l'ouverture du compartiment d'entrée 4 du ventilateur 1, comme illustré en Fig. 5 et Fig. 6.

L'air de refroidissement, après son réchauffement au contact des autres composants électroniques 3 du ventilateur médical 1, peut être évacué vers l'extérieur, c'est-à-dire vers l'atmosphère ambiante, via une (ou des) ouverture agencée au travers de la paroi de la carcasse 2 du ventilateur médical 1, comme illustré en Fig. 3, notamment via un ventilateur de refroidissement 22 illustré en Fig. 5.

Par ailleurs, afin de permettre d'enrichir l'air aspiré par la micro-soufflante, il est prévu une entrée d'oxygène 23, comme visible en Fig. 3, laquelle peut être reliée fluidiquement à une source d'oxygène gazeux, par exemple à une bouteille d'oxygène comprimé ou une canalisation d'oxygène faisant partie du réseau de canalisations de gaz d'un bâtiment hospitalier. Le mélange air/oxygène se fait préférentiellement en amont de la micro-soufflante, par exemple dans le caisson 14 avant son entrée dans la micro-soufflante.

La micro-soufflante est quant à elle une micro-soufflante classique comprenant un moteur électrique équipé d'un arbre ou axe rotatif portant une roue à ailettes agencée dans un compartiment à roue d'une volute. La roue est mobile en rotation dans le compartiment à roue en étant entraînée par le moteur électrique, pendant son fonctionnement, afin de délivrer un flux de gaz respiratoire (air ou air/O₂) dans le circuit de fourniture de gaz 13 terminé par le raccord de sortie de gaz 12.

Il est à noter que, selon un mode de réalisation particulier, le(s) filtre(s) pourrai(en)t être un (des) filtre(s) de type NRBC (Nucléaires, Radiologiques, Biologiques, Chimiques) permettant la protection du patient et de la machine dans cet environnement.

## Revendications

1. Ventilateur médical (1) comprenant une micro-soufflante motorisée et des composants électroniques (3), et comprenant en outre :
- un circuit d'air patient (10) reliant fluidiquement une entrée d'air patient (11) à une entrée d'aspiration de la micro-soufflante pour alimenter la micro-soufflante avec de l'air, et
- un circuit d'air de refroidissement (20) comprenant une entrée d'air de refroidissement (21) pour acheminer de l'air de refroidissement destinée à refroidir par échange thermique au moins une partie des composants électroniques (3),
et dans lequel l'entrée d'air patient (11) du circuit d'air patient (10) est équipée d'un premier filtre (31), et l'entrée d'air de refroidissement (21) du circuit d'air de refroidissement (20) est équipée d'un second filtre (32),
**caractérisé en ce que** :
- les premier et second filtres (31, 32) sont portés par une cassette de filtration (30) commune détachable,
- la cassette de filtration (30) est configurée pour se fixer, de manière détachable, au ventilateur (1) au niveau de l'entrée d'air patient (11) du circuit d'air patient (10) et de l'entrée d'air de refroidissement (21) du circuit d'air de refroidissement (20), et
- l'entrée d'air patient (11) du circuit d'air patient (10) et l'entrée d'air de refroidissement (21) du circuit d'air de refroidissement (20) sont situées dans au moins un compartiment d'entrée (4) ouvert vers l'extérieur, ledit compartiment d'entrée (4) étant configuré pour loger la cassette de filtration (30) comportant les premier et second filtre (31, 32).

2. Ventilateur selon la revendication 1, **caractérisé en ce que** le ventilateur (1) comprend une carcasse rigide, la cassette de filtration (30) étant configurée pour se fixer, de manière détachable, à la carcasse du ventilateur (1).

3. Ventilateur selon la revendication 1, **caractérisé en ce que** le compartiment d'entrée (4) comprend une paroi de séparation (5) interne définissant une première (5a) et une seconde chambre (5b), le premier filtre (31) venant se loger dans la première chambre (5a) et le second filtre (32) venant se loger dans la seconde chambre (5b), lorsque la cassette de filtration (30) est insérée dans le compartiment d'entrée (4).

4. Ventilateur selon la revendication 3, **caractérisé en ce que** la cassette de filtration (30) comprend un espacement (35) séparant les premier filtre (31) et second filtre (32), la paroi de séparation (5) interne du compartiment d'entrée (4) du ventilateur (1) venant se loger dans l'espacement (35) de la cassette de filtration (30), lorsque ladite cassette de filtration (30) est insérée dans le compartiment d'entrée (4) du ventilateur (1).

5. Ventilateur selon la revendication 1, **caractérisé en ce que** la cassette de filtration (30) comprend un cadre-support (34) portant les premier et second filtres (31, 32).

6. Ventilateur selon la revendication 1, **caractérisé en ce que** le circuit d'air de refroidissement (20) est configuré pour acheminer de l'air de refroidissement pour refroidir par échange thermique un ou plusieurs composants électroniques (3) choisis parmi une carte électronique à microprocesseur, un écran d'IHM, une batterie interne ou un moteur de micro-soufflante.

7. Ventilateur selon la revendication 1, **caractérisé en ce que** les premier et second filtre (31, 32) sont choisis pour stopper les polluants ayant une taille supérieure ou égale à 0.3 µm.

8. Ventilateur selon la revendication 1, **caractérisé en ce que** les premier et second filtres (31, 32) sont de type HEPA.

9. Ventilateur selon la revendication 1, **caractérisé en ce que** la cassette de filtration (30) commune détachable est configurée pour être montée et démontée depuis l'extérieur du ventilateur (1).

10. Ventilateur selon la revendication 1, **caractérisé en ce que** le cadre-support (34) de la cassette de filtration (30) comprend :
- une paroi externe (37) traversée par des orifices (38) de passage d'air,
- des moyens d'étanchéité fluidique (36) et
- des moyens de fixation configurés pour permettre une fixation de la cassette de filtration (30) au sein du ventilateur médical (1).

11. Ventilateur selon la revendication 1, **caractérisé en ce que** le premier filtre (31) a une taille supérieure à celle du second filtre (32).

12. Ventilateur selon la revendication 11, **caractérisé en ce que** le premier filtre (31) a une taille double de celle du second filtre (32).

## Patentansprüche

1. Medizinisches Beatmungsgerät (1), umfassend ein motorisiertes Mikrogebläse und elektronische Komponenten (3) und umfassend ferner:
- einen Patientenluftkreis (10), der fluidisch einen Patientenlufteinlass (11) mit einem Ansaugeinlass des Mikrogebläses verbindet, um das Mikrogebläse mit Luft zu versorgen, und
- einen Kühlluftkreis (20), umfassend einen Kühllufteinlass (21), um Kühlluft zu transportieren, die dazu bestimmt ist, durch Wärmeaustausch mindestens einen Teil der elektronischen Komponenten (3) zu kühlen,
und bei dem der Patientenlufteinlass (11) des Patientenluftkreises (10) mit einem ersten Filter (31) ausgestattet ist und der Kühllufteinlass (21) des Kühlluftkreises (20) mit einem zweiten Filter (32) ausgestattet ist,
**dadurch gekennzeichnet, dass**:
- der erste und der zweite Filter (31, 32) von einer lösbaren gemeinsamen Filterkassette (30) getragen werden,
- die Filterkassette (30) dazu ausgestaltet ist, lösbar am Beatmungsgerät (1) an dem Patientenlufteinlass (11) des Patientenluftkreises (10) und dem Kühllufteinlass (21) des Kühlluftkreises (20) befestigt zu werden, und
- der Patientenlufteinlass (11) des Patientenluftkreises (10) und der Kühllufteinlass (21) des Kühlluftkreises (20) in mindestens einem nach außen offenen Einlassraum (4) gelegen sind, wobei der Einlassraum (4) dazu ausgestaltet ist, die Filterkassette (30) aufzunehmen, die den ersten und den zweiten Filter (31, 32) enthält.

2. Beatmungsgerät nach Anspruch 1, **dadurch gekennzeichnet, dass** das Beatmungsgerät (1) ein starres Gehäuse umfasst, wobei die Filterkassette (30) dazu ausgestaltet ist, lösbar an dem Gehäuse des Beatmungsgeräts (1) befestigt zu werden.

3. Beatmungsgerät nach Anspruch 1, **dadurch gekennzeichnet, dass** der Einlassraum (4) eine innere Trennwand (5) umfasst, die eine erste (5a) und eine zweite Kammer (5b) definiert, wobei der erste Filter (31) in der ersten Kammer (5a) aufgenommen ist und wobei der zweite Filter (32) in der zweiten Kammer (5b) aufgenommen ist, wenn die Filterkassette (30) in den Einlassraum (4) eingesetzt ist.

4. Beatmungsgerät nach Anspruch 3, **dadurch gekennzeichnet, dass** die Filterkassette (30) einen Zwischenraum (35) umfasst, der den ersten Filter (31) und den zweiten Filter (32) trennt, wobei die innere Trennwand (5) des Einlassraums (4) des Beatmungsgeräts (1) in dem Zwischenraum (35) der Filterkassette (30) aufgenommen ist, wenn die Filterkassette (30) in den Einlassraum (4) des Beatmungsgeräts (1) eingesetzt ist.

5. Beatmungsgerät nach Anspruch 1, **dadurch gekennzeichnet, dass** die Filterkassette (30) einen Stützrahmen (34) umfasst, der den ersten und den zweiten Filter (31, 32) trägt.

6. Beatmungsgerät nach Anspruch 1, **dadurch gekennzeichnet, dass** der Kühlluftkreis (20) dazu ausgestaltet ist, Kühlluft zu transportieren, um durch Wärmeaustausch eine oder mehrere elektronische Komponenten (3) zu kühlen, die unter einer Leiterplatte mit Mikroprozessor, einem HMI-Bildschirm, einer inneren Batterie oder einem Mikrogebläsemotor ausgewählt sind.

7. Beatmungsgerät nach Anspruch 1, **dadurch gekennzeichnet, dass** der erste und der zweite Filter (31, 32) ausgewählt sind, um Schadstoffe mit einer Größe von 0,3 µm oder mehr zu stoppen.

8. Beatmungsgerät nach Anspruch 1, **dadurch gekennzeichnet, dass** der erste und der zweite Filter (31, 32) HEPA-Filter sind.

9. Beatmungsgerät nach Anspruch 1, **dadurch gekennzeichnet, dass** die lösbare gemeinsame Filterkassette (30) dazu ausgestaltet ist, von außerhalb des Beatmungsgeräts (1) montiert und demontiert zu werden.

10. Beatmungsgerät nach Anspruch 1, **dadurch gekennzeichnet, dass** der Stützrahmen (34) der Filterkassette (30) umfasst:
- eine Außenwand (37), die von Luftdurchlassöffnungen (38) durchquert wird,
- Mittel zur fluidischen Abdichtung (36) und
- Befestigungsmittel, die dazu ausgestaltet sind, eine Befestigung der Filterkassette (30) innerhalb des medizinischen Beatmungsgeräts (1) zu ermöglichen.

11. Beatmungsgerät nach Anspruch 1, **dadurch gekennzeichnet, dass** der erste Filter (31) eine Größe hat, die größer als die des zweiten Filters (32) ist.

12. Beatmungsgerät nach Anspruch 11, **dadurch gekennzeichnet, dass** der erste Filter (31) eine Größe hat, die doppelt so groß wie die des zweiten Filters (32) ist.

## Claims

1. Medical ventilator (1) comprising a motorized micro-blower and electronic components (3), and additionally comprising:
- a patient air circuit (10) fluidically connecting a patient air inlet (11) to a suction inlet of the micro-blower in order to supply the micro-blower with air, and
- a cooling air circuit (20) comprising a cooling air inlet (21) in order to convey cooling air which, by thermal exchange, is intended to cool at least some of the electronic components (3),
and in which the patient air inlet (11) of the patient air circuit (10) is equipped with a first filter (31), and the cooling air inlet (21) of the cooling air circuit (20) is equipped with a second filter (32),
**characterized in that**:
- the first and second filters (31, 32) are carried by a detachable common filtration cartridge (30),
- the filtration cartridge (30) is configured to fasten detachably to the ventilator (1) at the region of the patient air inlet (11) of the patient air circuit (10) and the cooling air inlet (21) of the cooling air circuit (20), and
- the patient air inlet (11) of the patient air circuit (10) and the cooling air inlet (21) of the cooling air circuit (20) are situated in at least one inlet compartment (4) open towards the outside, said inlet compartment (4) being configured to accommodate the filtration cartridge (30) having the first and second filters (31, 32).

2. Ventilator according to Claim 1, **characterized in that** the ventilator (1) comprises a rigid shell, the filtration cartridge (30) being configured to fasten detachably to the shell of the ventilator (1).

3. Ventilator according to Claim 1, **characterized in that** the inlet compartment (4) comprises an internal separation wall (5) defining a first chamber (5a) and a second chamber (5b), the first filter (31) being accommodated in the first chamber (5a) and the second filter (32) being accommodated in the second chamber (5b) when the filtration cartridge (30) is inserted into the inlet compartment (4).

4. Ventilator according to Claim 3, **characterized in that** the filtration cartridge (30) comprises a space (35) separating the first filter (31) and second filter (32), the internal separation wall (5) of the inlet compartment (4) of the ventilator (1) being accommodated in the space (35) of the filtration cartridge (30) when said filtration cartridge (30) is inserted into the inlet compartment (4) of the ventilator (1).

5. Ventilator according to Claim 1, **characterized in that** the filtration cartridge (30) comprises a support frame (34) carrying the first and second filters (31, 32) .

6. Ventilator according to Claim 1, **characterized in that** the cooling air circuit (20) is configured to convey cooling air in order, by thermal exchange, to cool one or more electronic components (3) chosen from among an electronic board with microprocessor, an HMI screen, an internal battery or a micro-blower motor.

7. Ventilator according to Claim 1, **characterized in that** the first and second filters (31, 32) are chosen to stop pollutants having a size of greater than or equal to 0.3 µm.

8. Ventilator according to Claim 1, **characterized in that** the first and second filters (31, 32) are HEPA filters.

9. Ventilator according to Claim 1, **characterized in that** the detachable common filtration cartridge (30) is configured to be mounted and unmounted from outside the ventilator (1).

10. Ventilator according to Claim 1, **characterized in that** the support frame (34) of the filtration cartridge (30) comprises:
- an outer wall (37) through which air passage orifices (38) extend,
- fluidic sealing means (36) and
- fastening means configured to allow the filtration cartridge (30) to be fastened inside the medical ventilator (1).

11. Ventilator according to Claim 1, **characterized in that** the first filter (31) has a size greater than that of the second filter (32).

12. Ventilator according to Claim 11, **characterized in that** the first filter (31) has a size twice that of the second filter (32).
